# EUROPEAN PATENT APPLICATION

(11) **EP 1 380 205 A1**
(43) Date of publication of application: **14.01.2004**
(21) Application number: 02759840.8
(22) Date of filing: 04.01.2002
(51) Int. Cl.: A01K 67/00, G01N 33/15, A61K 45/00, A61P 27/02

(54) **EXPERIMENTAL ANIMALS FOR EVALUATION OF THERAPEUTIC EFFECTS ON CORNEAL EPITHELIAL DAMAGES**

(30) Priority: 30.03.2001 JP 2001101250; 26.07.2001 JP 2001226460; 28.11.2001 JP 2001363147
(71) Applicant: Biochemical and Pharmacological Laboratories Inc., Tondabayashi-shi, Osaka 584-0023 (JP)
(72) Inventor: KATSUYAMA, Iwao, Biochem. & Pharmacol. Lab., Inc., Tondabayashi-shi, Osaka 584-0023 (JP)
(74) Representative: Schüssler, Andrea, Dr.
(86) International application number: PCT/JP2002/000007
(87) International publication number: WO 2002/080663

(57) **Abstract**

An excellent model animal having a corneal epithelial damage, an excellent method of evaluating a medicine using the model animal, and an excellent medicine selected by the evaluation method are provided. A model animal having a corneal epithelial damage, characterized in that the corneal epithelial damage is caused by contacting an ocular cornea of the animal with a water-absorbing material and thereby generating the difference in osmotic pressure between the inside and outside of corneal epithelium cells; a method of evaluating a medicine characterized by using the model animal; and a medicine selected by the evaluation method.

## Description

### TECHNICAL FIELD

This invention relates to a model animal having a corneal epithelial damage, a method of evaluating a medicine using the same, and a medicine selected by the method.

### BACKGROUND ART

The cornea is a very thin tissue of 0.52 to 1.0 mm thick, and constitutes together with the sclera a protective outer segment of the eyeball. The cornea has a simple and fine structure well organized into five layers: epithelium, Bowman's membrane, corneal stroma, Descemet's membrane and endothelium.

The term "corneal epithelial damage" means, in the literal sense, that corneal epithelium cells composing of the corneal epithelial layer, the forefront segments of the cornea, are damaged. There are a variety of causes for the corneal epithelial damages. Also, there are a variety of clinical findings of the damages. Depending on the presence or absence of ulcers in the basement membrane or corneal stroma, the corneal epithelial damages are classified into superficial punctate keratopathy (SPK), simple corneal erosion, recurrent corneal erosion, and corneal abrasion. The corneal epithelial damage may be caused by lacrimal fluid disorders, diseases of eyelids and/or conjunctiva, metabolic disorders, infections, trauma, drugs, and the like. Among them, in recent years, so-called dry eye which is a corneal epithelial damage caused by lacrimal fluid disorders has been drawing much attention, and dry eye patients, including potential dry eye patients, tend to increase in number.

The term "dry eye" is, in a few words, a general term of the damages which occur on the ocular surface, caused by quantitative or qualitative disorders of lacrimal fluid, or symptoms leading to such damages.

Lacrimal fluid plays an important role in maintaining a normal ocular function. Known roles of the lacrimal fluid are as follows: 1) it keeps keratoconjunctiva moist and protect it from being dried; 2) it keeps the surface of cornea smooth by forming a lacrimal fluid layer on the surface, and the lacrimal fluid layer functions as transparent optical refractive media; 3) antibacterial ingredients in lacrimal fluid prevent enter of viruses, bacteria and so on; 4) it flushes out foreign substances and/or wastes adhered to the surface of keratoconjunctiva; 5) it supplies oxygen, water and nutrients to cornea, one of a few tissues having no blood vessel therein; 6) it acts as a lubricant between the ocular surface and the eyelid on blinking or during an ocular movement; and 7) it functions as a migration path for a leukocyte in case of ocular trauma.

The surface of eyeball in a healthy condition is worn by lacrimal fluid. A layer of new lacrimal fluid (lacrimal fluid layer) is formed at every blinking, so that the ocular surface is protected from drying. While this lacrimal fluid layer begins to dry in approximately ten seconds, the sensory nerve distributed over the ocular surface detects drying and induces blinking, thereby keeping the ocular surface moistened with lacrimal fluid. This mechanism is known as a basal secretion of lacrimal fluid, and is distinguished from a reflex secretion, which occurs when sad or when dusts get into eye. The surface of keratoconjunctiva will get dry and be damaged in case the volume of lacrimal fluid decreases.

Dry eye has been called an ocular xerosis or a hypolacrimia, the most of which has been considered as one symptom of the Sjogren's syndrome. However, a type of the dry eye which impairs only the basal secretion without accompanying a Sjogren's syndrome, and a type of the dry eye due to the use of VDT (visual display terminal, or simply "display") are increasing in recent years.

In so-called visual information society of these days, the number of VDT workers is increasing in parallel with the growing use of OA apparatus including computers. The total number of dry eye patients in Japan now is estimated to be from one million to two million, and the number of patients tends to be increasing, including potential dry eye patients. The frequency of blinking will decrease when people continue watching the display (VDT) of OA apparatus for a long hour. Since lacrimal fluid is supplied to the ocular surface while the eye is closed by blinking, the decrease in the frequency of blinking leads to reduction in volume of lacrimal fluid, and is causative of corneal damages. This is because the prevalence of OA apparatus connects with the increase of dry eye patients. It is expected therefore that the number of dry eye patients will continue to increase with increasing opportunities for heavy burden to be imposed on eyes.

Besides the decrease in said blinking frequency brought about by the above VDT work, endogenous diseases such as Sjogren's syndrome, dry keratoconjunctivitis and Stevens-Johnson syndrome, and exogenous causes such as Cataract operation, medicines, trauma, wearing contact lens are also causative of dry eye due to the quantitative or qualitative deterioration of lacrimal fluid.

To date, various medicines for treatment of dry eye have been made available in the market. However, the existing medicines are unsatisfactory to meet with medical needs, and development of new medicines is still extensively in progress. In R & D of medicines, a system for evaluating the effects of medicine is essential, and the evaluation is made by tests using various model animals for dry eye.

Conventional model animals for dry eye include a rat Vitamin A-deficient model (Japanese Patent Laid-Open No. Hei 9-136832). In this model, a dry eye-like symptom is produced by breeding rats under Vitamin A-deficient conditions. As a matter of course, the symptom is healed by giving Vitamin A to said rats (oral or eye drop administration). Thus, these malnutritional model animals may be still unsuitable to evaluate medicines having various mechanisms of action.

As model animals having a corneal epithelial damage, there have been proposed model animals whose corneal epithelial damages are produced by treatment with iodine gas, or an organic solvent such as n-heptanol; by physical treatment with, for example, a knife; or by alkali corrosion treatment using, for example, sodium hydroxide.

Although these model animals may undoubtedly be a model animal having a corneal epithelial damage in that they have a damage in the corneal epithelium, they may be a problematic model animal because the mechanism of development of the damage is quite different from the physiological one. Further, the above-mentioned model animals have defects that the corneal epithelial damages produced by the above-mentioned methods are rapidly healed, which is inconvenient to evaluate medicines. Thus, these model animals cannot be a model animal having a prolonged corneal epithelial damage, which resists various medical treatments and ophthalmologists therefore have difficulty in the treatment, which has long been desired. (Teruo Nishida "Development of Medicine; vol. 9; Search for Medicine I", pp 287-291, published by Hirokawa Shoten, Tokyo (1990)).

Further, as a model animal having a corneal epithelial damage caused by disorders of lacrimal fluid (for example, a dry eye model), there has been reported a rabbit model which is produced by the compulsive eyelid retraction (rabbit compulsive eye lid retraction model) (Kei Nagano et al, New Ophthalmology, 13(2), pp 267-270(1996)). In the preparation of this model, the upper and lower eyelids of both eyes are everted under general anesthesia, are sutured, and are retracted compulsively for three hours. Air-drying during the long-term compulsive retraction deprives lacrimal fluid of water on the ocular surface, resulting in loss of the integrity of the lacrimal fluid layer, whereby a corneal epithelial damage is caused. The mechanism of development of the corneal epithelial damage in this model animal is that the lacrimal fluid layer on the ocular surface is destroyed over a long period of time, which is similar to the physiological mechanism of the natural dry eye. Therefore, this model animal can be more desirable than the Vitamin A-deficient model or the above-described model animals having a corneal epithelial damage, and is applicable to assay of medicines.

This model animal, however, has several defects that 1) it takes such a long time as 3 hours, to produce the model; 2) it is hard to constantly produce model animals (or eyeballs) having a symptom with a constant damage (severity); 3) the symptom is not uniform on the corneal surface; and 4) the damage is not maintained for a period of time sufficient to evaluate the therapeutic effect of medicines, as it is healed easily when the ocular surface is moistened with lacrimal fluid (because the healing power of a rabbit is much higher than that of a human). This model animal is more advantageous over the model animals which have been conventionally reported, but it may not yet be suitable and practical. Therefore, more excellent model animals have been desired to be provided.

As is clear from the above description, it is an important problem to be solved in the medical field to provide excellent model animals having a corneal epithelial damage, excellent methods of evaluating medicine using the same, and excellent medicines selected by the methods, and more excellent model animals, and such methods and medicines have been desired to be provided.

Therefore, the present inventors have conducted intensive studies in order to solve the above-described problem. As a result, the present inventors have found that the model animal of the present invention is useful as a model animal having a corneal epithelial damage, above all, a corneal epithelial damage caused by disorders of the lacrimal fluid, or so-called dry eye model; the method of producing a model animal of the present invention is useful; and the evaluation method using a model animal of the present invention is useful as a method of evaluating a medicine for treatment of a corneal epithelial damage. The present invention has been thus completed.

### DISCLOSURE OF INVENTION

The invention according to Claim 1 relates to a model animal having a corneal epithelial damage, characterized in that the corneal epithelial damage is caused by contacting an ocular cornea of said animal with a water-absorbing material and thereby generating the difference in osmotic pressure between the inside and outside of corneal epithelium cells.

The invention according to Claim 2 relates to the model animal as defined in Claim 1, characterized in that said corneal epithelial damage is dry eye.

The invention according to Claim 3 relates to the model animal as defined in Claim 1 or 2, characterized in that said model animal is a non-human mammalian or a fowl.

The invention according to Claim 4 relates to the model animal as defined in any one of Claims 1 to 3, characterized in that the water-absorbing material comprises at least one of materials selected from a polyol, a salt, an amino acid, a peptide and a water-soluble synthetic polymer.

The invention according to Claim 5 relates to the model animal as defined in Claim 4, characterized in that the water-absorbing material is a saccharide.

The invention according to Claim 6 relates to the model animal as defined in Claim 5, characterized in that the water-absorbing material comprises at least one of saccharides selected from the group consisting of glucose, maltose, sucrose, fructose, dextran and starch.

The invention according to Claim 7 relates to the model animal as defined in Claim 4, characterized in that the water-absorbing material comprises at least one of salts selected from an alkali metal salt and an alkaline earth metal salt.

The invention according to Claim 8 relates to the model animal as defined in any one of Claims 1 to 7, characterized in that the water-absorbing material is in the form of any one of powder, solution, gel, jelly and tablet.

The invention according to Claim 9 relates to the model animal as defined in any one of Claims 1 to 8, characterized in that the water-absorbing material is contacted with the ocular cornea through a water-permeable or semi-permeable product.

The invention according to Claim 10 relates to the model animal as defined in any one of Claims 1 to 9, characterized in that the model animal is rabbit.

The invention according to Claim 11 relates to the model animal as defined in any one of Claims 1 to 10, characterized in that the water-absorbing material is contacted with a portion of the ocular cornea.

The invention according to Claim 12 relates to the model animal as defined in any one of Claims 1 to 11, characterized in that the water-absorbing material is contacted with a pupil area of the ocular cornea.

The invention according to Claim 13 relates to the model animal as defined in Claim 12, characterized in that the water-absorbing material is contacted with the pupil area of the ocular cornea so that the contact area becomes a round shape.

The invention according to Claim 14 relates to the model animal as defined in Claim 13, characterized in that contact of the water-absorbing material is carried out by covering the ocular cornea with a water-impermeable thin membrane having a hole in its center part to place the hole on the pupil area, and contacting the water-absorbing material with the exposed site.

The invention according to Claim 15 relates to a method of evaluating a medicine for treatment of a corneal epithelial damage, characterized in that the method comprises the steps of:
contacting an ocular cornea of a model animal with a water-absorbing material and thereby generating the difference in osmotic pressure between the inside and outside of corneal epithelium cells to produce a symptom of the corneal epithelial damage;
administering a test medicine to the damaged ocular cornea; and
evaluating the degree of the symptom ameliorated in the ocular corneal epithelial damage site.

The invention according to Claim 16 relates to the method as defined in Claim 15, characterized in that said corneal epithelial damage is dry eye.

The invention according to Claim 17 relates to the method as defined in Claim 15 or 16, characterized in that the step of evaluating the degree of the symptom ameliorated in the corneal epithelial damage site is a step of evaluation by expressing the change in the stained area in terms of numerical values.

The invention according to Claim 18 relates to the method as defined in any one of Claims 15 to 17, characterized in that the model animal as defined in any one of Claims 1 to 14 is used.

The invention according to Claim 19 relates to the method as defined in any one of Claims 15 to 18, characterized in that the test medicine is an eye drop.

The invention according to Claim 20 relates to a medicine for treatment of a corneal epithelial damage, which is selected by the method as defined in any one of Claims 15 to 19.

### BRIEF DESCRIPTION OF THE DRAWINGS

Fig. 1 is a schematic view of a square thin membrane used in producing the model animal example 1.
Fig. 2 is a schematic view of a circular thin membrane used in producing the model animal example 2.
Fig. 3 is a graph showing a sequential change of the damaged corneal area according to Example 4.
Fig. 4 is a graph showing a sequential change of the healing rate according to Example 4.
Fig. 5 is a graph showing a sequential change of the damaged corneal area according to Example 5.
Fig. 6 is a graph showing a sequential change of the healing rate according to Example 5.
Fig. 7 is a photographic image of a typical eyeball of the model animal example 2, which was taken immediately after the model animal was made.
Fig. 8 is a H-E staining image (photographic magnifying power of 100) of a corneal section of the model animal example 2, which was taken immediately after the model animal was made.
Fig. 9 is a H-E staining image (photographic magnifying power of 400) of a corneal section of the model animal example 2, which was taken immediately after the model animal was made.
Fig. 10 is a H-E staining image (photographic magnifying power of 100) of a corneal section of the non-treated eyeball.
Fig. 11 is a H-E staining image (photographic magnifying power of 400) of a corneal section of the non-treated eyeball.
Fig. 12 is a graph showing a sequential change of the damaged corneal area according to Example 6.
Fig. 13 is a graph showing a sequential change of the healing rate according to Example 6.
Fig. 14 is a graph showing a sequential change of the damaged corneal area according to Comparative Example 3.
Fig. 15 is a photographic image of a typical eyeball of the comparative model animal example 1, which was taken immediately after the model animal was made.
Fig. 16 is a H-E staining image (photographic magnifying powder of 100) of a corneal section of the comparative model animal example 1, which was taken immediately after the model animal was made.
Fig. 17 is a H-E staining image (photographic magnifying power of 400) of a corneal section of the comparative model animal example 1, which was taken immediately after the model animal was made.
Fig. 18 is a graph showing a sequential change of the damaged corneal area according to Comparative Example 4.

### BEST MODE FOR CARRYING OUT THE INVENTION

The term "corneal epithelial damage" in this invention means that the integrity of the corneal epithelial layer (the forefront segment of the cornea) is lost due to any impairment.

The term "dry eye" in this invention refers to corneal epithelial damages which are caused by endogenous diseases such as ocular xerosis, Sjogren's syndrome and Stevens-Johnson syndrome, or exogenous causes such as surgical operation, medicines, trauma, wearing contact lens, or symptoms accompanying the corneal epithelial damage.

The water-absorbing material for use in producing model animals having the corneal epithelial damage in this invention may be any material which reduces moisture on the eyeball and which is pharmacologically acceptable.

The phrase, "reduce moisture on the eyeball," means that water on the ocular surface is absorbed and, in addition, the difference in osmotic pressure is generated between the inside and outside of the ocular corneal epithelium cells, thereby inducing leakage of water from the corneal epithelium cells to decrease the intracellular moisture content.

The term "pharmacologically acceptable" in this invention means that the effective pharmacological properties of the medicine to be evaluated are not influenced (the pharmacological properties are not inhibited or enhanced).

In the present invention, any water-absorbing material can be used to produce the model animal having the corneal epithelial damage insofar as the material has the above-mentioned properties. Preferable specific examples of the water-absorbing materials include polyols, salts, amino acids, peptides, water-soluble synthetic polymers, and the like.

Examples of the polyols include polyalcohols such as glycerin and ethylene glycol, monosaccharides such as glucose, galactose, mannose and fructose, oligo saccharides such as sucrose, lactose, maltose and cyclodextrin, poly-saccharides such as starch, glycogen, dextran, pullulan and chitosan, and sugar alcohols such as glucitol, galactitol, mannitol and xylitol.

Examples of the salts include organic alkali metal salts such as sodium acetate, sodium citrate, potassium acetate and potassium citrate, inorganic alkali metal salts such as sodium chloride and sodium sulfate, organic alkaline earth metal salts such as calcium acetate, magnesium acetate, calcium citrate and magnesium citrate, and inorganic alkaline earth metal salts such as calcium chloride, magnesium chloride, potassium sulfate, magnesium sulfate, and the like.

Examples of the amino acids include glycine, alanine, valine, leucine, isoleucine, serine, threonine, aspartic acid, glutamic acid, asparagine, glutamine, lysine, hydroxylysine, arginine, cysteine, methionine, phenylalanine, tyrosin, tryptophane, histidine, proline, hydroxy proline and their derivatives.

Examples of the peptides include low molecular peptides such as glutathione, proteins such as albumin, globulin, collagen and gelatin, their partial protein hydrolysates and derivatives.

Examples of water-soluble synthetic polymers include polyethylene glycol, polyethylene oxide, sodium polystyrenesulfonate, polyvinyl alcohol, polyvinylpyrrolidone and the like.

The above-mentioned water-absorbing material may be used alone or as a composition comprising two or more of any water-absorbing materials. Furthermore, as described herein below, the water-absorbing material may be used by blending with excipients, extenders, thickeners and so forth, as required.

The model animals in the present invention may be any animal of which eyeball can be used for the evaluation of medicine, and are not particularly limited. For example, any of non-human mammalian such as monkey, dog, cat, rabbit, guinea pig, rat, mouse, cow, sheep, pig and goat, as well as fowls such as chicken, domestic duck, quail and ostrich, can be used. Mammalians such as monkey, dog, cat, rabbit, guinea pig, rat, mouse, cow, sheep, pig and goat, are preferable model animals, for the reason that model animals having a given property are constantly produced and that they are easy to breed or treat during the experiment. Especially, rabbit, cat, dog, pig and goat are more preferable, for the reason that the size of their eyeballs is suitable for the experiment.

The conditions for breeding the animals before the production of model animals are not particularly limited, and may be chosen in usual ways. Each animal should preferably be bred under the same conditions in order to obtain uniform model animals.

The model animals according to the present invention may be any animal which is suitably used for evaluating the therapeutic effect of a test medicine. The animals can be used, which are in any form. One embodiment of making such model animal of the invention is, for example, shown below.

An animal for production of model animal is anesthetized, and its eyelids are kept open. The eyelids can be kept open in conventional manners, for example, by using an adhesive or an eyelid retractor. Then, a water-absorbing material is contacted with the ocular surface for an appropriate period of time, after which the water-absorbing material is removed from by, for example, washing the ocular surface, to obtain a model animal having a corneal epithelial damage.

The water-absorbing material may be contacted directly or indirectly with the surface of cornea, without any particular limitation. When the water-absorbing material is contacted with the surface of the cornea, the water-absorbing material may cover the whole surface of ocular cornea or only a portion of said surface. The position or size of the ocular cornea where the water-absorbing material contacts, may vary depending on the kind of animal to be chosen, and the like. To evaluate the therapeutic effect of a test medicine by expressing it in terms of numerical values, it is preferable that the size of the contact area is the same as that of the pupil.

In contacting the water•absorbing material with a portion of the ocular surface, the contact area may be in any geometric shape and the contact position may be in any position according to the purpose, appropriate conditions can be properly set. The shape includes circle, ellipse, square, rectangle, triangle, and polygons including star. The shape and the position may be chosen as desired depending on the purposes. Among them, to evaluate the therapeutic effect of a test medicine by expressing it in terms of numerical values, it is preferable that the shape of the contact area is in the round shape and that the contact position is on the pupil area.

The water-absorbing material may be in any physical state. For example, the material can be used in the form of powder, solution, gel, jelly or tablet, depending on the physical properties of said material or composition comprising the material to be used.

In order to evaluate the therapeutic effect of a medicine conveniently and precisely, it is preferable to obtain the results as numerical values to permit comparative evaluation. In order to obtain the results to be expressed in terms of numerical values, it is preferable that the water-absorbing material of a pre-selected size is contacted with the eyeball in a pre-selected area. In order to obtain the contact area with a pre-selected size and a pre-selected area, it is preferable to employ a method of: (1) covering the cornea with a water-impermeable thin membrane having in it a hole of an appropriate shape, and placing the water-absorbing material on the water-impermeable thin membrane; or (2) mounting a cylindrical tube on the cornea, and placing the water-absorbing material into the tube. In both cases, a suitable material which is water-impermeable and pharmacologically acceptable can be appropriately selected for the thin. membrane or cylindrical tube to be used, without any particular limitation. Examples of the material include synthetic resins such as nylon, polyethylene, polypropylene, polystyrene, polycarbonate, vinyl chloride and polyfluoroethylene fibers, and metals such as aluminum, titanium and stainless steel, tree, bamboo and the like. For the purpose of obtaining the experimental results with the least possible errors, it is usually preferable to avoid using materials which allow the body fluid component of eyeball to permeate.

Contact of the water-absorbing material with the ocular surface may usually be carried out by putting said material directly on the ocular surface; but said material can be contacted indirectly with the ocular corneal surface, e.g., through a product made of a water-permeable material, depending on the purpose. The material for the product is not particularly limited insofar as it is water-permeable or semi-permeable. Examples of the material include sugar-based polymer materials such as cellulose or its derivatives, synthetic polymer-based materials such as polyvinyl alcohol, and the like. Also, the shape of the product is not particularly limited insofar as the desired object can be attained. The shape of the product is preferably in the form of thin membrane, for the reason that, for example, the properties intrinsically possessed by the water-absorbing material can be utilized and its handling is easy. The method of contacting the water-absorbing material through the product made of a water-permeable or semi-permeable material has an advantage that the water-absorbing material is conveniently removed after the treatment.

In addition, according to the present invention, the corneal epithelial damage can be produced all over the ocular surface. Alternatively, a corneal epithelial damage with a pre-selected area and a uniform symptom can be made at a pre-selected area on the ocular surface by contacting the water-absorbing material with a portion of the eyeball. The latter method enables us to evaluate still more conveniently and precisely the therapeutic effect of a medicine, for the reason that the change in the symptom can be easily measured quantitatively as the change in the surface area of the site. For example, in one preferred embodiment of the present invention, the model animal having a corneal epithelial damage can be suitably produced by preparing a water-impermeable thin membrane having in its center part a hole of an appropriate shape such as circle, covering the ocular surface with said water-impermeable thin membrane so that the hole in it comes on around the pupil area of the ocular surface with the eyelid kept open, and contacting the water-absorbing material with the exposed ocular surface.

As described above, the water-absorbing material in the present invention has a function to dehydrate ocular surface, and at the same time, induce leakage of water from the ocular corneal epithelium cells by generating the difference in osmotic pressure between the inside and outside of the cells, to cause a model animal to exhibit a symptom of the corneal epithelial damage. To obtain suitable model animals having a corneal epithelial damage, the difference in osmotic pressure between the inside and outside of the ocular corneal epithelium cells is preferably not less than 0.5, more preferably not less than 1, still more preferably not less than 2, and especially preferably not less than 3. When the water-absorbing material in the physical state of, e.g., powder, contacts with the ocular surface, it absorbs water on said surface and is dissolved in the water to form a solution. The solution of said material formed on the ocular surface is a thick solution close to saturation and therefore generates the difference in osmotic pressure between the inside and outside of the cell membranes of the corneal epithelium cells. Since the cell membranes have properties similar to those of a semi-permeable membrane, upon generation of the difference in osmotic pressure, a force to eliminate the difference is produced by water which permeates to the outside of the cells through the cell membranes to dilute the hypertonic solution. The permeated water to dilute the hypertonic solution further serves to dissolve the remaining water-absorbing material, so that the concentration of the solution outside the cells is still kept at a high level and, therefore, the difference in osmotic pressure is not eliminated. Moreover, since water is continuously permeates from the inside of the cells, leakage of water from the inside of the cells will occur continuously.

The difference in osmotic pressure between the inside and outside of the cells varies mainly depending on the kind of the water-absorbing material to be used. For example, a saturated lactose solution has an osmotic pressure ratio of about 2, and a saturated sodium chloride solution has an osmotic pressure ratio of about 20. Since the intracellular fluid of corneal epithelium has an osmotic pressure of about 1, the difference in osmotic pressure is calculated to be about 1 when a saturated lactose solution is contacted with the corneal epithelium cell, and the difference is about 19 when a saturated sodium chloride solution is contacted with the corneal epithelium cell. The difference in osmotic pressure generated when the water-absorbing material is contacted with the cells is not strictly consistent with the difference in osmotic pressure between the saturated solution of a water-absorbing material and the intracellular fluid, but it is thought to be useful as a reference.

Incidentally, the osmotic pressure ratio, as used herein, refers to a value of the ratio of the osmotic pressure of a sample to that of a physiological saline solution.

In the present invention, the water-absorbing material may be used as a composition prepared by appropriately formulating excipients, extenders, thickeners and so forth with the material in order to adjust its osmotic pressure within a suitable range. Such excipients, extenders and thickeners can be also used to adjust the shape and the physical properties of the water-absorbing material when contacted to the ocular corneal. The kind of the excipients, extenders, and thickeners usable for such purposes is not particularly limited. For instance, crystalline cellulose, carboxymethylcellulose, carboxymethylethylcellulose, hydroxypropylcellulose, hydroxypropylmethylcellulose, wheat flour, wheat starch, wheat germ powder, rice powder, rice starch, potato starch, corn starch, corn starch granulation, carboxymethyl starch, hydroxypropyl starch, dextran, dextrin, agar, pectine, macrogol, gelatin and the like, can be appropriately used according to the purpose.

The amount of the water-absorbing material to be contacted with the ocular surface is not particularly limited, and may be appropriately chosen so as to obtain the desired model animals.

The degree (severity) of the symptoms of the model animal used for the evaluation of medicine should appropriately be adjusted, depending on the purposes for evaluating the therapeutic effect of a test medicine described below. This adjustment can readily be done by changing the kind, composition, amount, contact area, contact time, etc. of the water-absorbing material when the model animal of the invention is used, but the adjustment is not easy when the conventional compulsive eyelid retraction models are used.

The water-absorbing material may be contacted with the corneal surface for a period of time required to obtain the desired model animals, without any particular limitation. Typically, the contact time is preferably 5 to 60 minutes, more preferably 10 to 40 minutes, and especially preferably 15 to 25 minutes. When the contact time is shorter than 5 minutes, pathologic conditions with a severity which can be used as an evaluation system are not obtained in most cases. When the contact time is longer than 60 minutes, no further severe pathologic conditions are obtained in most cases. In many cases, the contact time of 15 to 25 minutes is most desirable to obtain uniform pathologic conditions with an appropriate severity.

After the treatment with the water-absorbing material for a preset time, the water-absorbing material is removed from the ocular surface. When the water-impermeable thin membrane, instruments such as a cylindrical tube, the product made of a water-permeable or semi-permeable material, or the like is used, they are also removed from the ocular surface. The water-absorbing material is removed as much as possible by a method of, for example, washing the ocular surface with a suitable amount of a pharmacologically acceptable liquid. Examples of the pharmacologically acceptable liquid include a physiological saline solution and an isotonic phosphate buffer solution.

In the model animal of the present invention, the corneal epithelium is made dry by dehydration and shows the symptom of epithelial abrasion. Without medical treatment etc., this symptom will not be easily recovered even if the ocular surface is moistened with lacrimal fluid, and the pathological conditions last for a long period of time, for example, 10 hours or more, in some cases, dozens of hours. Therefore, the therapeutic effect of medicines can be stably evaluated.

Such characteristics of the model animal according to the present invention are of significant importance in view of a fact that the corneal surface of the conventional compulsive eyelid retraction model (three hour retraction) is simply kept dried with substantially no epithelial cell abrasion, such a symptom recovers in about an hour owing to secretion of lacrimal fluid, and thus said model has some difficulty in the use for evaluation of the therapeutic effect of medicine.

The present invention is also characteristic in that the animal model of the present invention can be prepared in approximately 20 minutes (usually within about 60 minutes) after the eyelid retraction, while the production of the conventional compulsive eyelid retraction model requires 3 hours or longer.

In the conventional compulsive eyelid retraction models, there have been unsolved technical problems that 1) it is difficult to evaluate quantitatively the size of the corneal epithelial damage and the degree of the symptom due to uneven size and degree because the entire ocular surface is dried; 2) it is difficult to evaluate the therapeutic effect of a medicine because natural healing by lacrimal fluid proceeds rapidly, so that the time for the evaluation is short; and the like. The present invention is further characteristic in that since the animal model of the present invention has a pre-selected size of the corneal epithelial damage with a uniform severity on the corneal surface, the severity or the change with time of the symptom can be expressed in terms of numerical values by simply measuring the surface area of the damaged site, which enables us to make a quantitative evaluation.

The "test medicine" to be evaluated by the method using the model animal of the present invention is not particularly limited, and may be any one of those which are intended to be evaluated for the therapeutic effect on the corneal epithelial damages. Compositions comprising at least one active ingredient prepared for the purpose of treating corneal epithelial damages are encompassed in the test medicine to be evaluated. Among them, taking into consideration the mechanism of producing the model animal of the present invention, compositions prepared for the purpose of treating a corneal epithelial damage caused by disorders of the lacrimal fluid, so-called dry eye, are preferable as test medicines to be evaluated.

Usually, the test medicine to be evaluated refers to drugs and quasi drugs. The test medicine also includes supplemental foods such as neutraceuticals (functional foods), specified dietary supplements and health care foods, including, for instance, those which has at least one action of serving to protect corneal epithelial damages, promoting the recovery of corneal injuries, alleviating mucous membrane stimulus or protect mucous membrane. The specific examples include hyaluronic acid, chondroitin sulfate, dextran sulfate, adrenal cortical hormone, and the like. As a matter of course, not only medicines having a known mechanism of action, but also medicines having a new mechanism of action, can be evaluated.

The medicine to be evaluated is usually administered locally in the form of eye drop to the eyeball. But, medicines for any administration route can be evaluated, without any particular limitation. The medicines for both parenteral and oral administrations can be evaluated. Examples of medicines for parenteral administration include, in addition to the above-mentioned eye drop, ointment for eye, injection, medicines for external use, suppository and the like. The dose of the medicine is not particularly limited. The dose should be determined *ad libitum* based on the conditions such as the properties of the medicines, the kind, age or body weight of the subject animal to be administered, and the optimal dose is appropriately decided.

Conditions for administration such as timing and number of times thereof may also be appropriately decided so as to be optimal depending on the properties of the medicine, the purposes of experiments and evaluations, and the like, and are not particularly limited. For example, with reference to the timing of administration in the case of evaluating eye drop, in most cases is used a method which comprises administering eye drop for a few times every 30 minutes immediately after the model animal is prepared, and for a few times every one hour thereafter, and observing and evaluating the change in the symptom after each administration. The number of times of administration varies depending on the properties of the medicine, and may be once or more, preferably not less than 2 times, more preferably 4 to 6 times or more.

The method of evaluating a test medicine using the model animals of the present invention may be carried out in any manner, insofar as the desired object can be attained. For example, the evaluation method of the present invention can be carried out by observing reflection of light on the ocular surface, or by observing tissues on the ocular surface with a microscope.

In addition, in order to quantitatively evaluate the effect of a medicine by expressing it in terms of numerical values, usually, it is preferred that the size of the area of ocular corneal epithelial damages is measured by staining the ocular cornea after administration of the test medicine, and the size of the damaged area is compared with the size of area of the ocular corneal epithelial damages obtained in a group to which no test medicine is administered, or with the size of the area of said damages measured before administration of the test medicine (hereinafter referred to as the stained area comparison method). Usually, as the damaged area is stained with dyes, the use of this method is advantageous in that the effect of administration of the medicine can be evaluated in terms of the changes in the stained area, thereby evaluating the effect of the medicine quantitatively. The potency of a medicine can be determined as percent decrease in the stained area.

Any pharmacologically acceptable dyes can be used for the stained area comparison method. Preferably used are a fluorescein salt, rose bengal, sulfarodamine B, and lissamine green which are used for vital stain of the ocular surface, and it is more preferable to use a fluorescein salt.

A method to measure the size of the stained area is not particularly limited, and a most suitable method may be chosen depending on the kind of the dye, and the like. For example, when a fluorochorome such as fluorescein sodium is employed therein, the size of the stained area can be expressed in terms of numerical values by measuring fluorescence intensity, or by processing and analyzing the image data of the ocular surface taken by digital camera and the like.

One of specific examples of evaluating the therapeutic effect of a test medicine using a model animal of the present invention is shown below.

The ocular surface of the model animal is well washed with a physiological saline solution, and an appropriate concentration (e.g., 2%) of an aqueous sodium fluorescein solution is instilled to the surface to stain the damaged area of the cornea. The ocular surface is again well washed with the physiological saline solution to remove free dye. The picture of the ocular surface is taken by digital camera. The image data thus obtained is processed by the use of an image-analyzing software, whereby the size of the stained area (the damaged area of the cornea) is expressed in terms of the number of pixels. After administration of a medicine, the size of the damaged area of the cornea is expressed in terms of the number of pixels in the same manner. The degree of amelioration can be determined by obtaining the ratio of the size of the stained area after administration of the medicine to that before administration of the medicine.

Further, according to the present invention, the therapeutic effect of a test medicine can also be evaluated, for instance, by 1) measuring the time required to heal the damages provided that the dose number is kept constant, or 2) counting the dose numbers to heal the damages provided that the test medicine is administered at constant intervals, besides by determining the area as described above.

### EXAMPLES

In the following examples, the present invention is more specifically described by the Examples of Production of the model animal and Examples of evaluation of medicine. Incidentally, the present invention is not limited to the model animals, the methods, and the like, descried in the following Examples, and includes all the model animals, the methods, and the like, encompassed by the appended Claims.

### Example 1: Production of the model animal example 1

Rabbits (New Zealand white, male, 13 weeks old, about 3 kg body weight) were selected as animals to be tested, and quarantined and habituated.

They were bred in animal-breeding boxes set in a homothermal and homohumid room (room temperature of 21 ± 3°C; relative humidity of 50 ± 20%, 12 hours illumination (7 a.m. light-up, 7 p.m. lights-out); and 10-15 times ventilation/hour). They were freely given a feed (manufactured by Oriental Yeast Co., Ltd; RC4) and water (given after sterilizing tap water by filtering it through the membrane having pore size of 0.2 µm in diameter).

The animals were anaesthetized (general anesthesia) by intramuscular injection of 20 mg/kg of ketamine hydrochloride (manufactured by Sankyo Co., Ltd under the trade name "Ketaral 50 for intramuscular injection") and 10 mg/kg of xylazine hydrochloride (manufactured by Bayer under the trade name "Seraktal 2% for injection to dog and cat"). The eyelids of the animal were kept open and glued with the adhesive for surgery (manufactured by Sankyo Co., Ltd. under the trade name "Aron alpha A (Sankyo)").

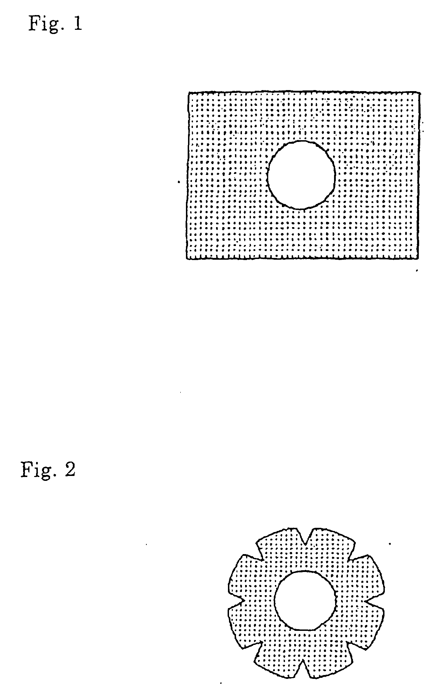

Each one of the eyeballs was covered with a Teflon sheet film (20 x 30 mm, 0.15 mm thick; Fig. 1) having a hole of 8 mm ϕ in size at around the center thereof.

Ninety one parts by weight of powder sugar (manufactured by Sankyo Shokuhin Co., Ltd; a mixture of sucrose and corn starch (97:3; w/w)) and 9 parts by weight of distilled water were kneaded together to give a clayey composition, which was selected as a water-absorbing material. 0.5 g of the composition was placed on the opening of the film covering the eyeball, and kept contacted uniformly with the ocular surface for 20 minutes.

Thereafter, the film and the composition were removed from the ocular surface. Said ocular surface was well washed with a sufficient amount of a physiological saline solution, whereby the model animal example 1 was obtained.

### Example 2: Production of the model animal example 2

A model animal was produced by using powder sugar (manufactured by Sankyo Shokuhin Co., Ltd.; a mixture of sucrose and corn starch (97:3; w/w) as the water-absorbing material.

A quarantined and habituated animal (rabbit) to be tested was anaesthetized (general anesthesia) in the same manner as in Example 1, and further anaesthetized locally by instillation of an eye drop containing a 0.4% oxybuprocaine hydrochloride (manufactured by Senju Pharmaceutical Co., Ltd. under the trade name "Anerocurl"). Then, eyelid retraction was done using a Vanguard eyelid retractor.

The eyeball was covered with a Teflon sheet film (21 mm ϕ, 0.2 mm thick; Fig. 2) having a hole of 8 mmϕin size at around the center thereof.

One gram of the powder sugar (manufactured by Sankyo Shokuhin Co., Ltd.; a mixture of sucrose and corn starch (97:3; w/w)) was used as the water-absorbing material, and placed on the opening of the film covering the eyeball, and kept contacted uniformly with the ocular surface for 20 minutes. In case lacrimal fluid was leaked while the eyeball was kept contacted with the film, an appropriate amount of the powder sugar was added as appropriate.

Thereafter, the film and the water-absorbing material were removed from the ocular surface. Said ocular surface was thoroughly washed with a sufficient amount of a physiological saline solution, whereby the model animal example 2 was obtained.

### Example 3: Production of the model animal example 3

A model animal was produced in the same manner as in Example 2 by using grinded sodium chloride as the water-absorbing material.

As the water-absorbing material, 1 g of the sodium chloride having a particle size of 150 µm or less in diameter after grinding (sieved by using a standard sieve (Sieve No. 100) of Japanese Industrial Standard) was used. The water-absorbing material was placed on the opening of the film covering the eyeball, and kept contacted uniformly with the ocular surface for 20 minutes.

Thereafter, the film and the water-absorbing material were removed from the ocular surface, and then the ocular surface was thoroughly washed with a sufficient amount of a physiological saline solution, whereby the model animal example 3 was obtained.

### Comparative Example 1: Production of the comparative model animal example 1

A quarantined and habituated animal (rabbit) to be tested was anaesthetized in the same manner as in Example 1, and the eyes were kept open for 3 hours by way of eyelid retraction using a Vanguard eyelid retractor. The comparative model animal example 1 was thereby obtained.

### Comparative Example 2: Production of the comparative model animal example 2

A model animal was prepared in the same manner as in Example 2 by using 1 g of a water-absorbing polymer instead of powder sugar.

As the water-absorbing polymer, powdery acrylic acid polymer, obtained from sanitary napkin "Sofy Active Support" (manufactured by Unicharm Corporation) was used. The water-absorbing polymer was placed on the opening of the film covering the eyeball, and kept contacted uniformly with the ocular surface for 20 minutes.

Thereafter, the film and the water-absorbing polymer were removed from the ocular surface. Said ocular surface was well washed with a sufficient amount of a physiological saline solution, whereby the comparative model animal example 2 was obtained.

### Example 4: Evaluation of medicine with the model animal example 1

This test was carried out by the use of the model animal 1 for the purpose of confirming that the model animal of the present invention shown in Example 1 (model animal example 1) and the method for the evaluation of the present invention are adequate as an assay system to evaluate medicines for treating corneal epithelial damages.

### [Administration of a test medicine]

A pharmaceutical formulation containing 0.1% sodium hyaluronate (made available by Santen Pharmaceutical Co., Ltd. under the trade name "Hyalein 0.1"; hereinafter referred to as "Hyalein") was used as a test medicine.

The tests were carried out with respect to the following four groups, using four eyeballs (right and left eyeballs of two animals) per group. About 50 µL/dose (one drop instilled via a commercially available container corresponds to about 50 µL) of "Hyalein" was instilled for Groups ① to ③, and a physiological saline solution was instilled, instead of "Hyalein", for Group ④.
Group ①: Instillation of drop was carried out at 30 minutes intervals for 0 to 6 hours, at an hour intervals for 6 to 12 hours, at 2 hours intervals up to 24 hours and thereafter at 4 hours intervals.
Group ②: Instillation of drop was carried out at 30 minutes intervals for 0 to 6 hours, at 2 hours intervals for 6 to 24 hours and thereafter at 4 hours intervals.
Group ③: Instillation of drop was carried out at 4 hours intervals from 6 hours after the model animal was prepared.
Group ④: Instillation of drop was carried out at 30 minutes intervals for 0 to 6 hours, at an hour intervals for 6 to 12 hours, at 2 hours intervals up to 24 hours and thereafter at 4 hours intervals.

Incidentally, administration of the medicine was discontinued when the recovery of the corneal damages was confirmed based on the data obtained. The animal was considered to have recovered from the corneal damage when the size of the stained area of the cornea (the damaged area of the cornea) was 0 according to the experiments mentioned below.

### [Collection of Area Data]

The damaged area of the cornea was stained by instillation of 50 µL of an aqueous 2% sodium fluorescein solution onto the ocular surface in each group before the administration of medicine and at each preset time point. The ocular surface was washed with a sufficient amount of a physiological saline solution, and the photograph thereof was taken by a digital camera. The data obtained was analyzed by an image-analyzing software (made available by Mitani Corporation under the trade name "Win ROOF"), and the size of the stained area (the damaged area of the cornea) was counted as the number of pixels. Incidentally, the data were collected at each preset time point before each instillation.

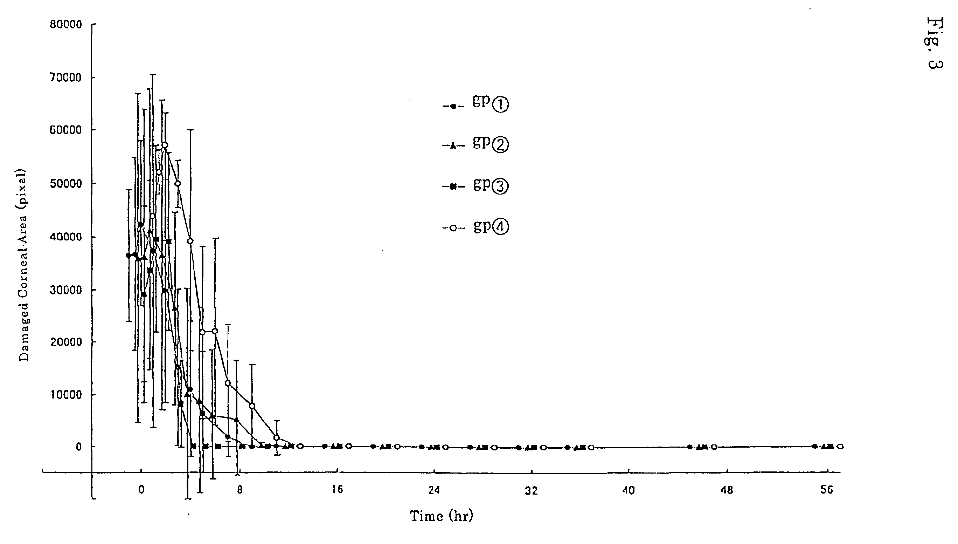

The sequential changes in the damaged area of the cornea are shown in Table 1 and Fig. 3.

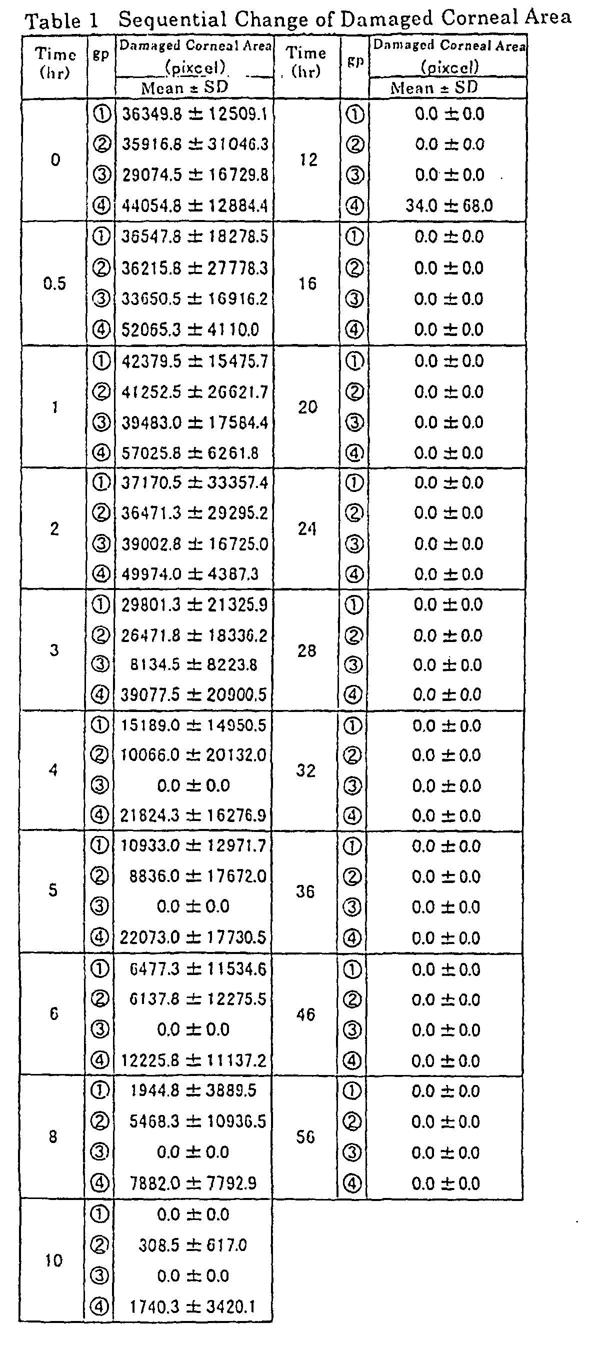

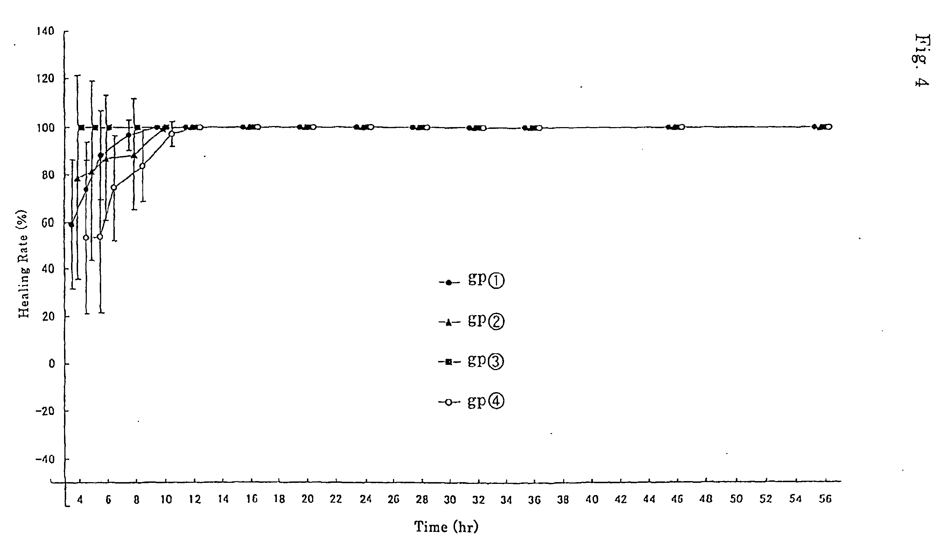

The sequential change of the healing rate is shown in Table 2 and Fig. 4.

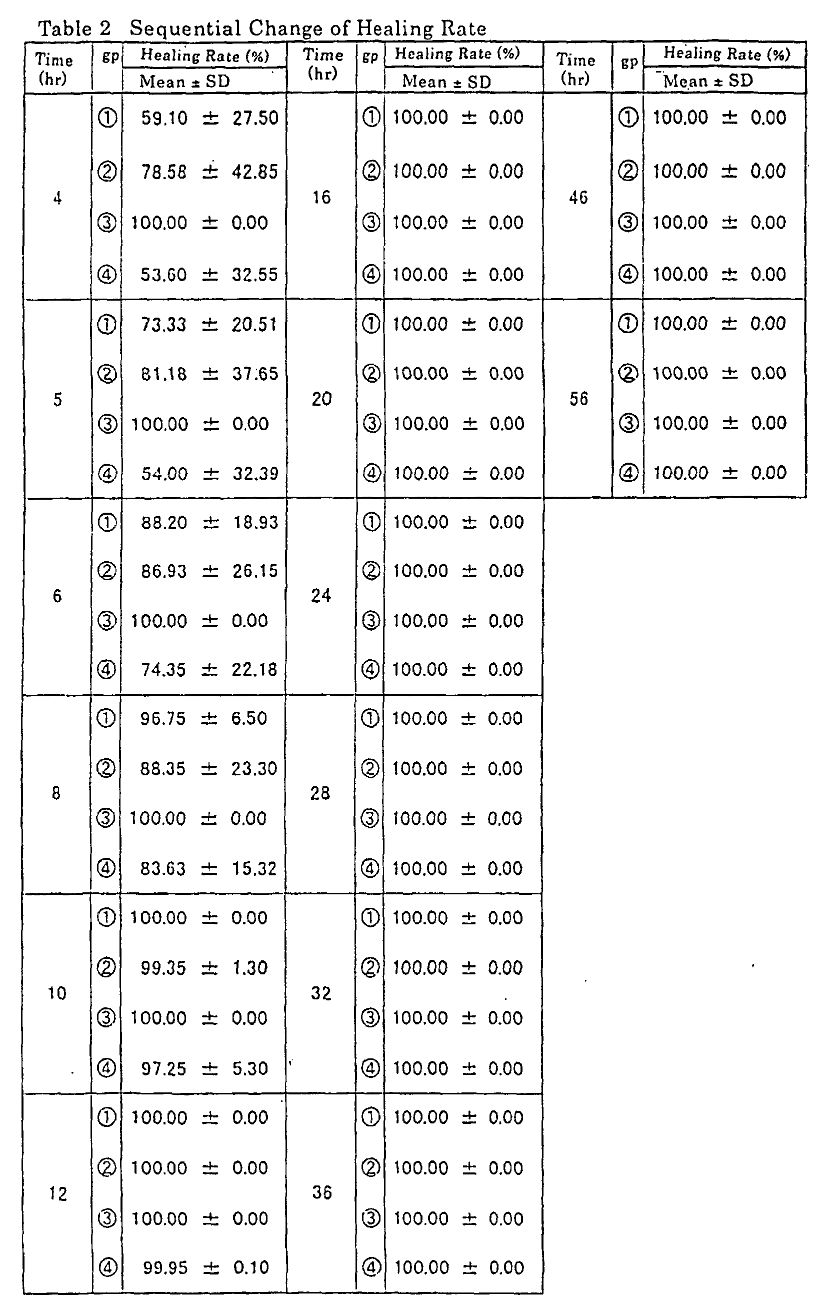

### [Data Analysis]

As understood from Tables 1 & 2 and Figs 3 & 4, the model animals of the present invention were characterized in that the corneal damages thereof gradually worsened after the model animals were prepared, and the animals began to recover after the damages reached the maximum level. Therefore, the evaluations were made by using as a reference the size of the damaged area of the cornea at a time when the size reached the maximum level.

In this test, based on the results shown in Tables 1 and 2 and Fig. 3, evaluations were made by using as the reference value ("Size as Reference for Evaluation") the size of the damaged area of the cornea which was measured 3 hours after the model animal was prepared.

The size of the area (the number of pixels) stained by sodium fluorescein was defined as the size of the damaged area of the cornea, and the Healing Rate (%) was calculated according to the following formula 1: $\text{(Formula 1)} \text{Healing Rate (%) = (SB - SP) / SB x 100}$
SB: Size as Reference for Evaluation (the number of pixels)
SP: Size of Stained Area (the number of pixels) measured at each time point of data collection

The results described above indicate that the model animal of the present invention, method of producing model animals of the present invention and method for the evaluation of the invention were adequate as an assay system to evaluate medicines for treating corneal epithelial damages.

### Example 5: Evaluation of medicine with the model animal example 2

This test was carried out by the use of the model animal 2 for the purpose of confirming that the model animal of the present invention shown in Example 2 (model animal example 2) and the method for the evaluation of the present invention are adequate as an assay system to evaluate medicines for treating corneal epithelial damages.

### [Administration of a test medicine]

"Hyalein" was used as a test medicine.

The tests were carried out with respect to the 4 groups, using four eyeballs (right and left eyeballs of two animals) per group, as described in Example 4. About 50 µL/dose of "Hyalein" was instilled for Group ① to ③, and a physiological saline solution was instilled instead of "Hyalein", for Group ④.

The test medicine was administered in the same manner as in Example 1.

As in Example 4, administration of the medicine was discontinued when the recovery of the corneal damages was confirmed based on the data obtained. The animal was considered to have recovered from the corneal damage when the size of the damaged area of the cornea was 0.

### [Collection of Area Data]

The area data were collected in the same manner as in Example 4.

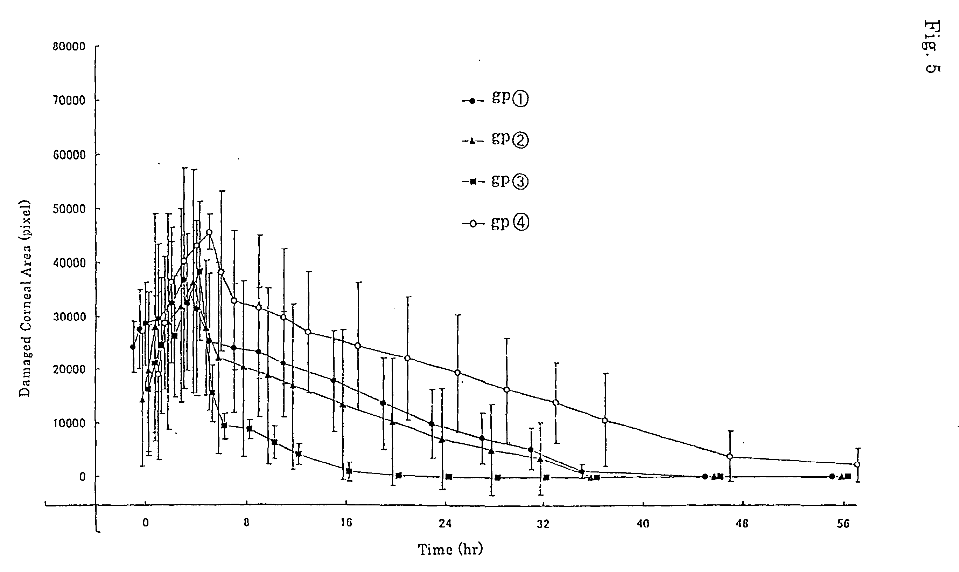

The sequential change of the damaged area of the cornea is shown in Table 3 and Fig. 5.

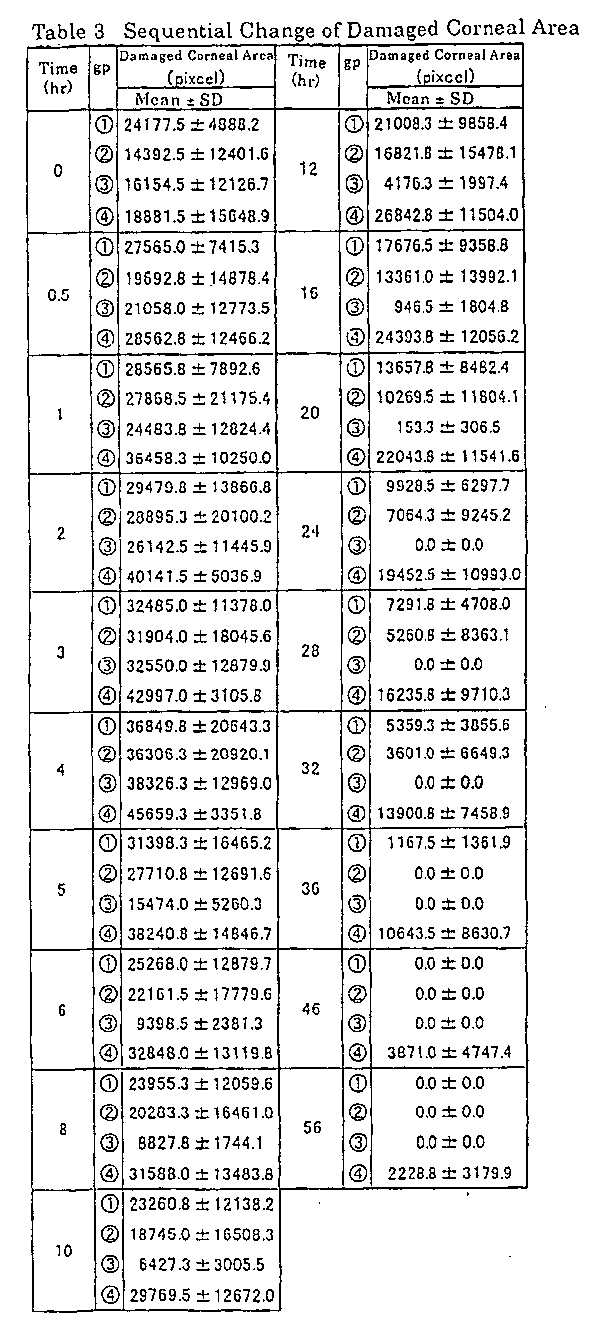

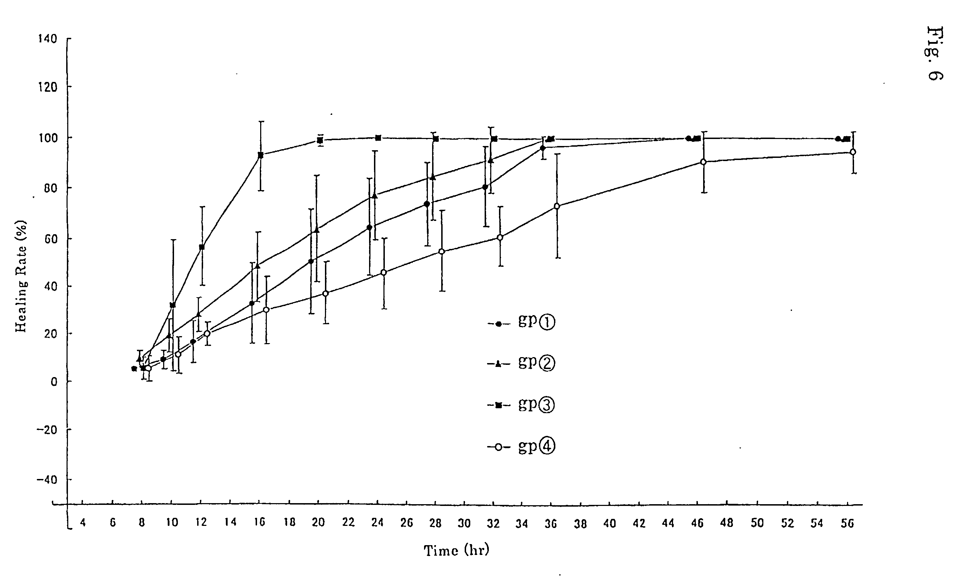

The sequential change of the healing rate is shown in Table 4 and Fig. 6.

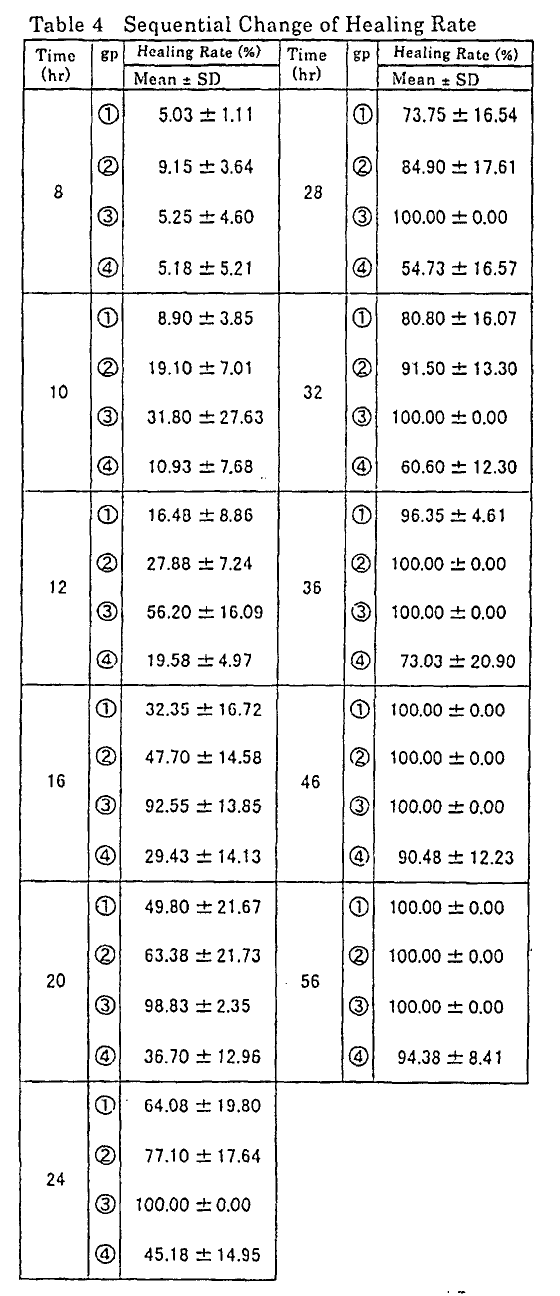

### [Data Analysis]

The data were analyzed in the same manner as in Example 4.

In this test, based on the results shown in Table 3 and Fig. 5, the Healing Rate was calculated using as the reference value the size of the damaged area of the cornea which was measured 6 hours after the model animal was prepared. The formula 1 was used for said calculation.

The results described above indicate that the model animal of the present invention, methods of producing model animals of the present invention and method for the evaluation of the invention were adequate as an assay system to evaluate the therapeutic effects of medicines for treating corneal epithelial damages.

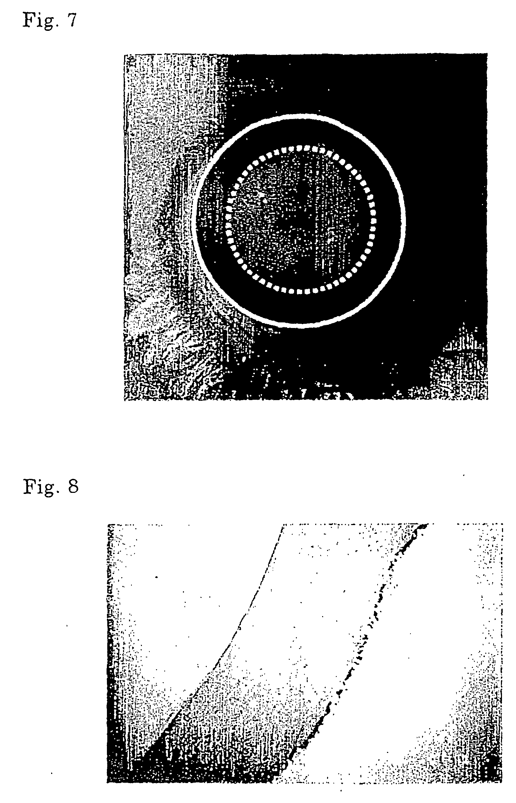

The photographic image of a typical eyeball, which was taken by a digital camera immediately after the model animal was prepared (0 hr), is shown in Fig. 7.

It can be seen that uniform corneal epithelial damages were formed in a round shape (uniform area stained with sodium fluorescein was formed in a round shape) on the ocular surface of the model animal example 2.

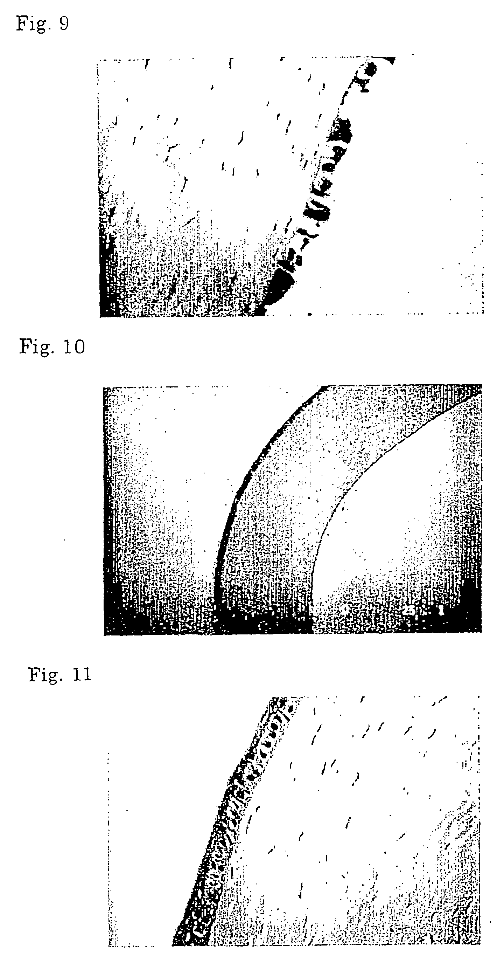

The photographic images of corneal sections stained with hematoxylin-eosin (H-E), which were taken immediately after the model animal was prepared (0 hr), are shown in Fig. 8 (photographic magnifying power of 100) and Fig. 9 (photographic magnifying power of 400). Just for comparison, the photographic images of corneal sections of an untreated eyeball stained with H-E are also shown in Fig. 10 (photographic magnifying power of 100) and Fig. 10 (photographic magnifying power of 400).

It could be confirmed that the uniform layer of epithelium cells were formed on the surface of cornea of said untreated eyeball, while the layer of the corneal epithelium cells immediately after the model animal example 2 was prepared were widely injured and abraded, and a corneal epithelial damage was caused on a tissue level.

### Example 6: Evaluation of medicine with the model animal example 3

This test was carried out by the use of the model animal 3 for the purpose of confirming that the model animal of the present invention shown in Example 3 (model animal example 3) and the method for the evaluation of the present invention are adequate as an assay system to evaluate medicines for treating corneal epithelial damages.

### [Administration of a test medicine]

"Hyalein" was used as a test medicine.

The tests were carried out with respect to the 4 groups, using four eyeballs (right and left eyeballs of two animals) per group, as described in Example 4 and 5. About 50 µL/dose of "Hyalein" was instilled for Group ① to ③, and a physiological saline solution was instilled instead of "Hyalein", for Group ④.

The test medicine was administered in the same manner as in Example 1.

As in Example 4 and 5, administration of the medicine was discontinued when the recovery of the corneal damages was confirmed based on the data obtained. The animal was considered to have recovered from the corneal damage when the size of the damaged area of the cornea was 0.

### [Collection of Area Data]

The area data were collected in the same manner as in Example 1.

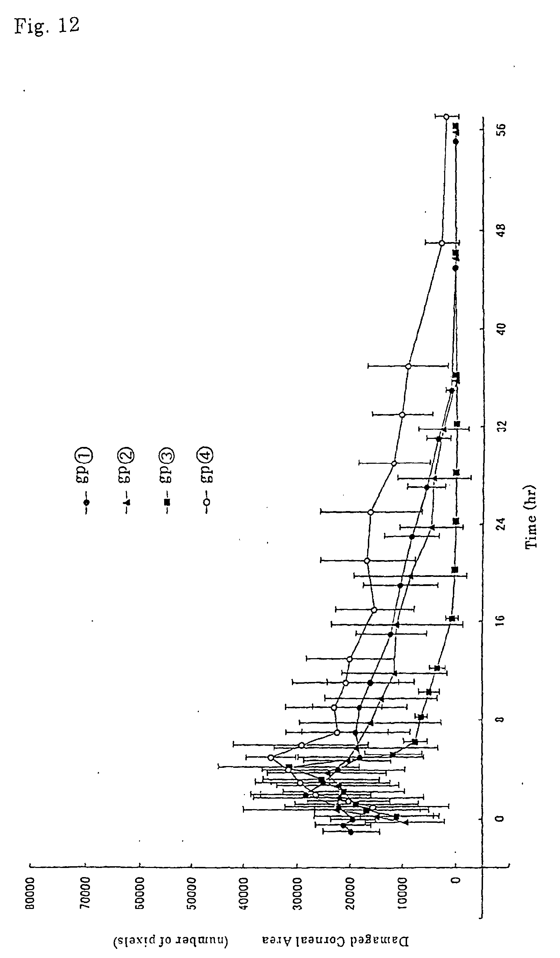

The sequential change of the damaged area of the cornea is shown in Table 5 and Fig. 12.

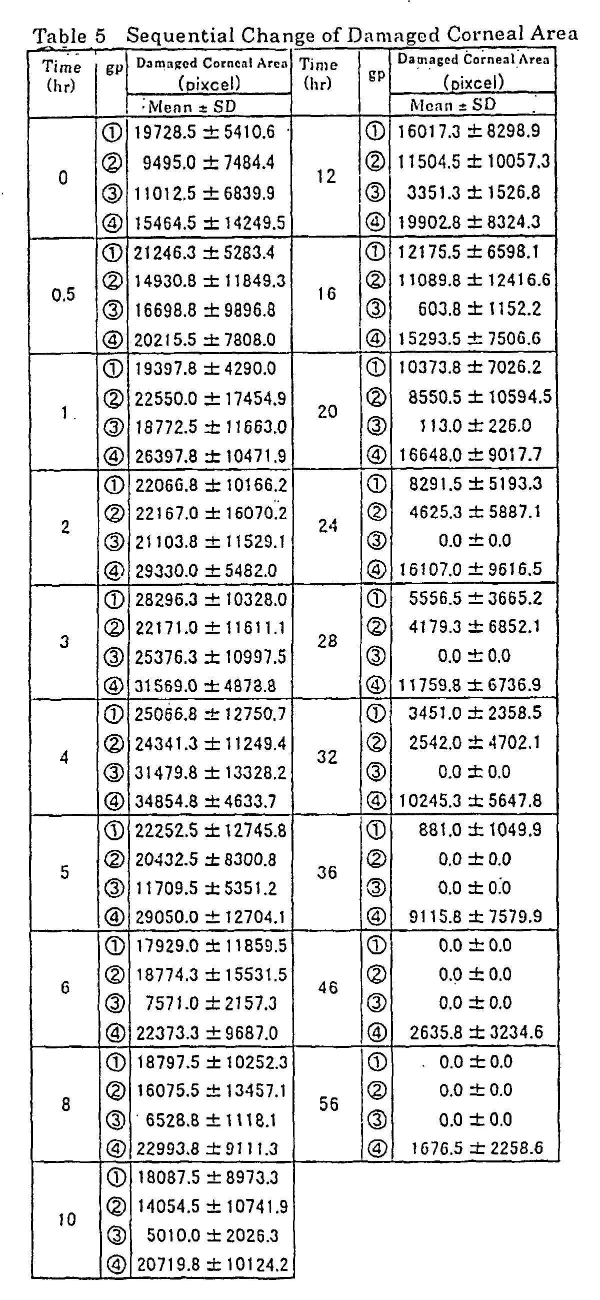

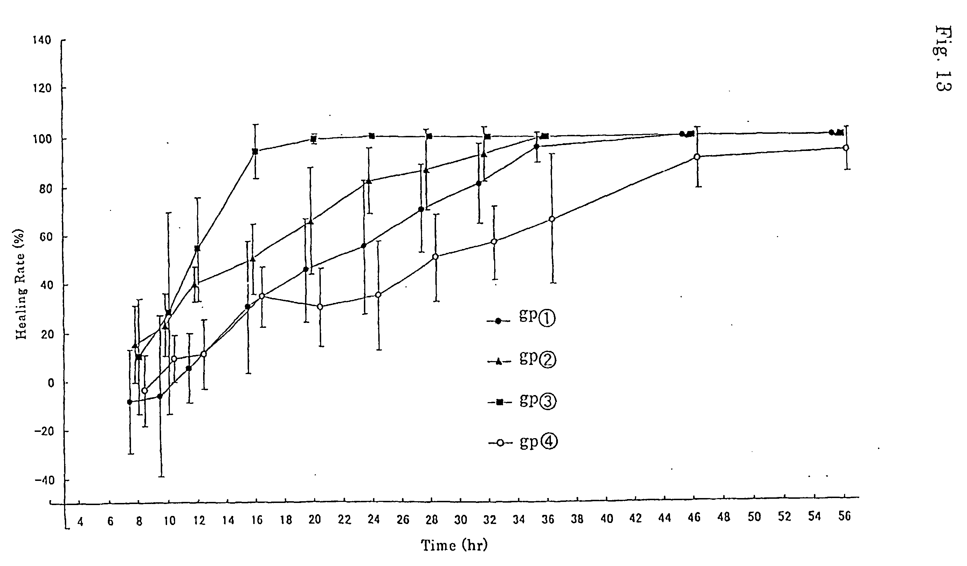

The sequential change of the healing is shown in Table 6 and Fig. 13.

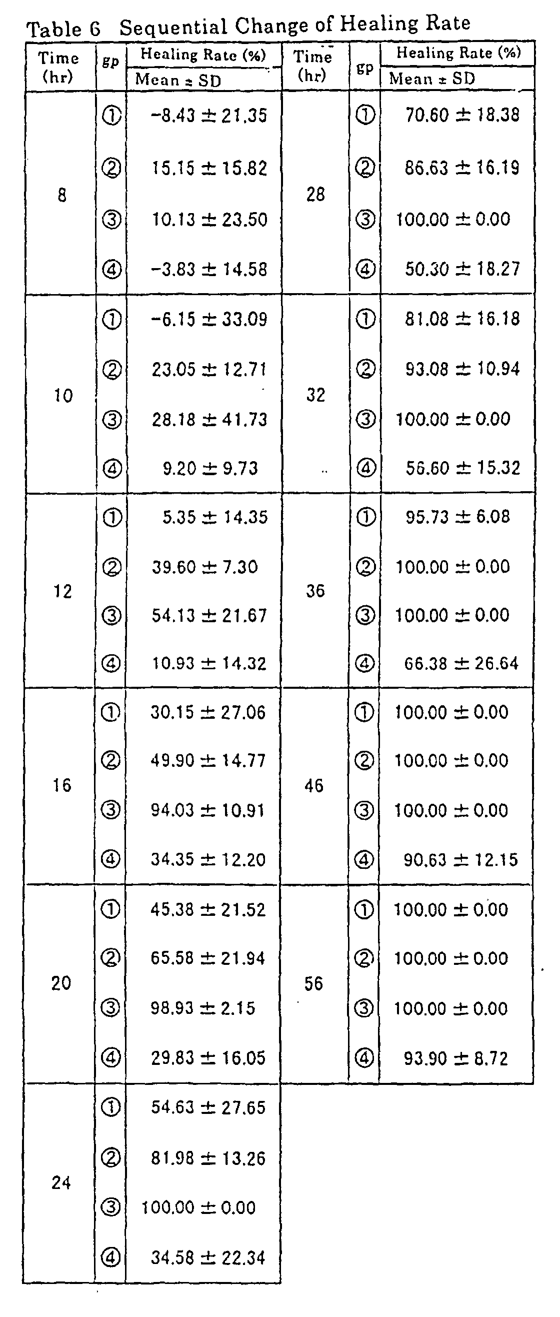

### [Data Analysis]

The data were analyzed in the same manner as in Example 4 and 5.

In this test, based on the results shown in Table 5 and Fig. 12, the Healing Rate was calculated using as the reference value the size of the damaged area of the cornea which was measured 6 hours after the model animal was prepared. The formula 1 was used for said calculation.

The results described above indicate that the model animal of the present invention, methods of producing model animals of the present invention and method for the evaluation of the invention were adequate as an assay system to evaluate the therapeutic effects of medicines for treating corneal epithelial damages.

### Comparative Example 3: Evaluation of medicine with the comparative model animal example 1

The tests were carried out in the same manner as in Examples 4 to 6 by the use of the comparative model animal example 1 shown in Comparative Example 1.

### [Administration of a test medicine]

"Hyalein" was used as a test medicine.

The tests were carried out with respect to the 4 groups, using four eyeballs (right and left eyeballs of two animals) per group, as described in Example 4 to 6. About 50 µL/dose of "Hyalein" was instilled for Group ① to ③, and a physiological saline solution was instilled instead of "Hyalein", for Group ④.

The test medicine was administered in the same manner as in Examples 4 to 6.

As in Examples 4 to 6, administration of the medicine was discontinued when the recovery of the corneal damages was confirmed based on the data obtained. The animal was considered to have recovered from the corneal damage when the size of the damaged area of the cornea was 0.

### [Collection of Area Data]

The area data were collected in the same manner as in Examples 4 to 6.

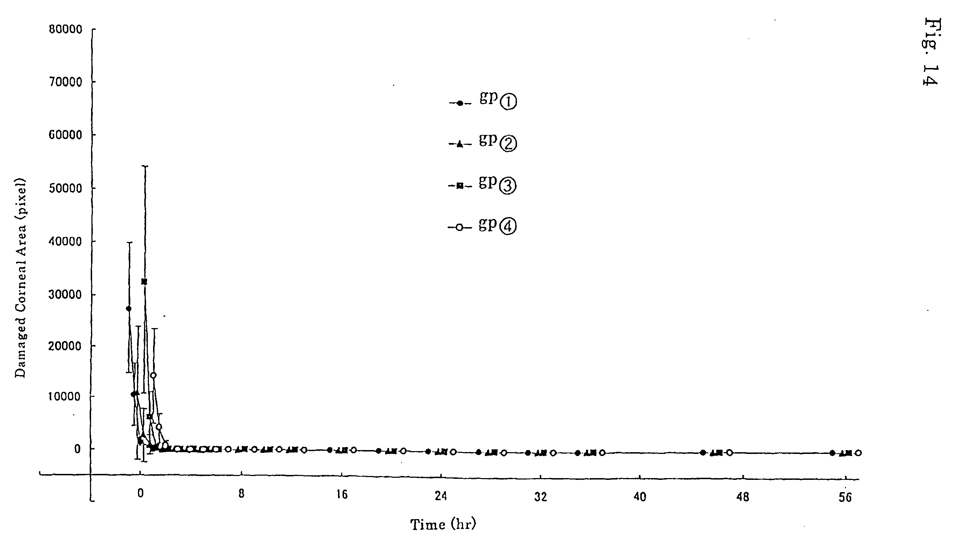

The sequential changes in the damaged area of the cornea are shown in Table 7 and Fig. 14.

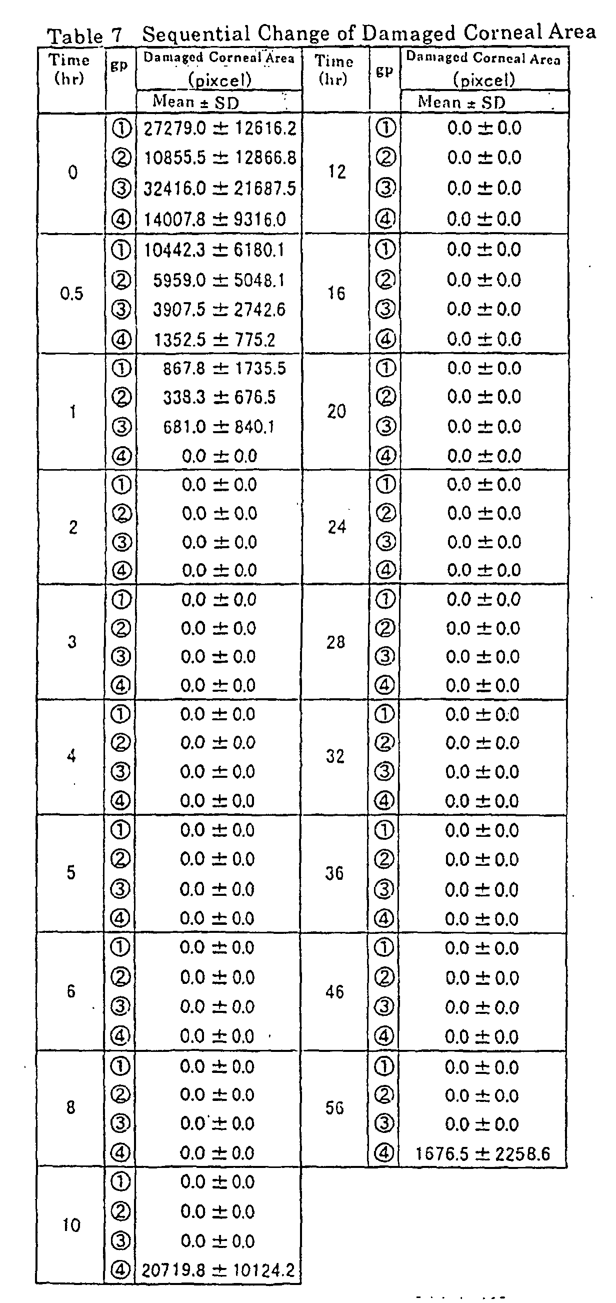

### [Data Analysis]

As understood from Table 7 and Fig. 14, unlike the model animal of the invention (model animal examples 1 to 3), the comparative model animal example 1 is characterized in that it began to recover from the disease immediately after the model animal was prepared.

The above results indicate that: 1) the healing rate of the corneal damage in the comparative model animal example 1 was too fast; and 2) therefore the therapeutic effect, if any, of a test medicine could hardly be detected when evaluated using the comparative model animal example 1.

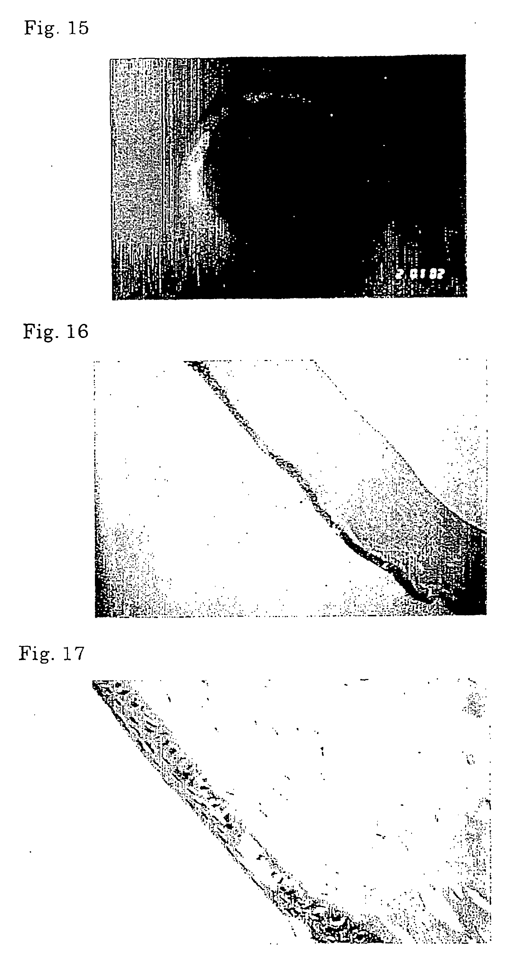

The photographic image of a typical eyeball, which was taken by a digital camera immediately after the comparative model animal example 1 was prepared (0 hr), is shown in Fig. 15.

It is seen that the corneal epithelial damages were un-uniformly formed (the area was un-uniformly stained with sodium fluorescein) on the ocular surface of the comparative animal model example 1. When compared to those observed in Fig. 7 of Example 5, the difference is obvious.

The photographic images of corneal sections stained with H-E, which were taken immediately after the comparative model animal example 1 was prepared (0 hr), are shown in Fig. 16 (photographic magnifying power of 100) and Fig. 17 (photographic magnifying power of 400).

In the cornea immediately after the comparative model animal example 1 was prepared, it was observed that the layer of the corneal epithelium cells were damaged and abraded, and confirmed that a corneal epithelial damage was caused on a tissue level. However, as compared to those observed in Fig. 8 and Fig. 9 of Example 5, is can be seen the difference is obvious, and the corneal damages caused are less severe and non-uniform relative to the model animal example 2.

In the comparative model animal example 1, the corneal damages were caused by air-drying the corneal surface by compulsive eyelid retraction for a long time, to dehydrate the corneal surface. It is presumed that severe damages cannot be caused on the corneal epithelium by this method of preparing model animal because water outside the cornea can be removed but water inside the corneal epithelium cells cannot effectively be removed. Thus, it is presumed that the damages themselves were healed rapidly.

### Comparative Example 4: Evaluation of medicine with the comparative model animal example 2

The tests were carried out in the same manner as in Examples 4 to 7 by the use of the comparative model animal example 2 shown in the Comparative Example.

### [Administration of a test medicine]

"Hyalein" was used as a test medicine.

The tests were carried out with respect to the 4 groups, using four eyeballs (right and left eyeballs of two animals) per group, as described in Examples 4 to 7. About 50 µL/dose of "Hyalein" was instilled for Groups ① to ③ and a physiological saline solution was instilled instead of "Hyalein", for Group ④.

The test medicine was administered and the data were obtained in the same manner as in Examples 4 to 7.

As in Examples 4 to 7, administration of the medicine was discontinued when the recovery of the corneal damages was confirmed based on the data obtained. The animal was considered to have recovered from the corneal damage when the size of the damaged area of the cornea was 0.

### [Collection of Area Data]

The area data were collected in the same manner as in Examples 4 to 7.

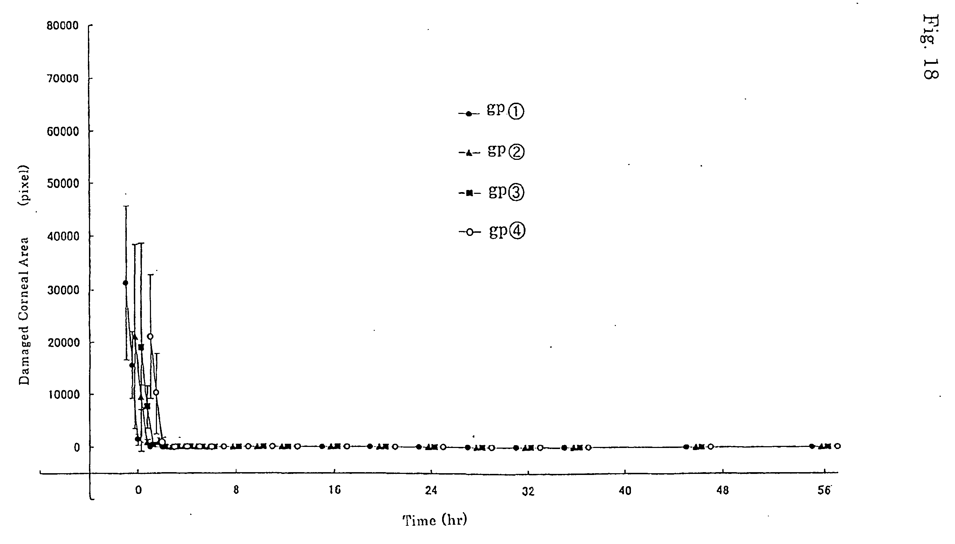

The sequential change of the damaged area of the cornea is shown in Table 8 and Fig. 18.

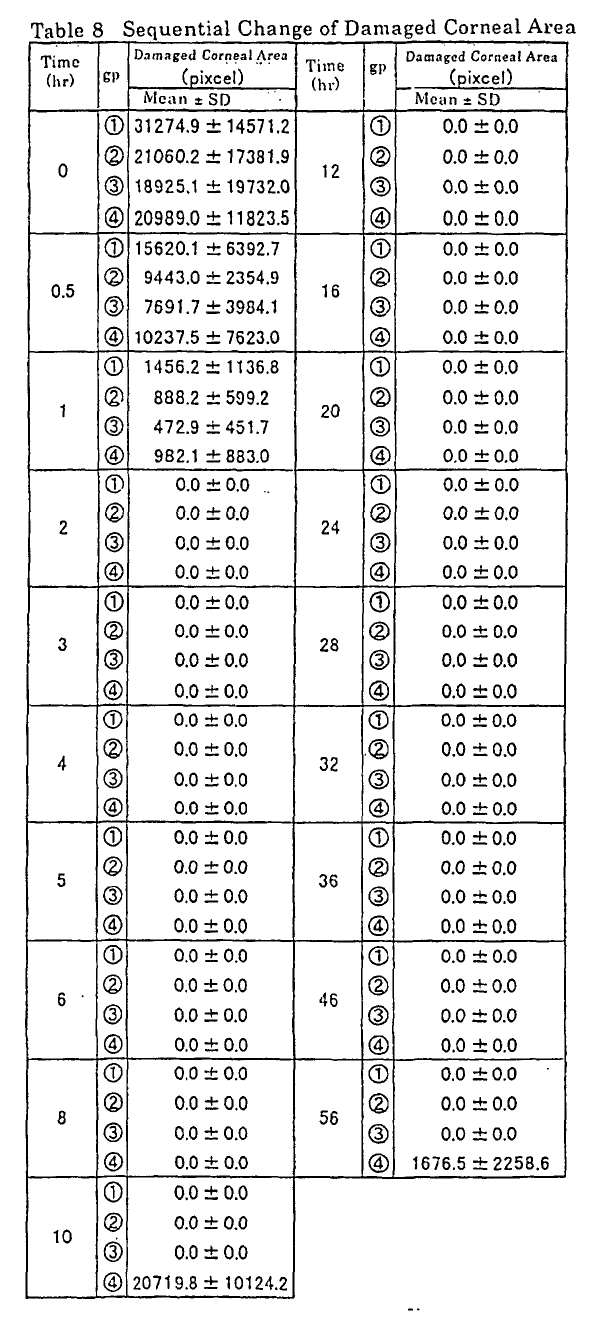

### [Data Analysis]

As understood from Table 8 and Fig. 18, like the comparative model animal example 1, the comparative model animal example 2 is characterized in that it began to recover from the disease immediately after the model animal was prepared.

The above results indicate that: 1) the healing rate of the corneal damage in the comparative model animal example 2 was too fast; and 2) therefore the therapeutic effect, if any, of a test medicine could hardly be detected when evaluated using the comparative model animal example 2.

In the comparative model animal example 2, the corneal damages were caused by contacting a water-absorbing material with the corneal surface, to dehydrate the corneal surface. It is presumed that sever damages cannot be caused on the corneal epithelium by this method of preparing model animal, as in the comparative model animal example 1, because water outside the cornea can be rapidly removed but water inside the corneal epithelium cells cannot effectively be removed. Thus, it is presumed that the damages themselves were also healed rapidly in this example, as in the comparative model animal example 1.

It can be seen from the aforementioned Examples 1 to 6 and Comparative Examples 1 to 4 that:
(1) the model animals of the invention (model animal examples 1 to 3) can maintain the damages for a long period of time (not less than 10 hours) so that it is easy to evaluate test medicines, whereas the comparative model animal examples 1 and 2 are recovered from the corneal damages in a short period of time (about 1 hour) so that it is difficult to evaluate the therapeutic effects of test medicines;
(2) the model animals of the invention give rise to corneal damages with a uniform shape and severity on the ocular surface (Fig. 7 of Example 5), whereas a conventional model animal (comparative model animal example 1) gives rise to corneal damages with a non-uniform shape and severity (Fig. 15 of Comparative Example 3);
(3) severe corneal damages (abrasion and damage in the layer of the corneal epithelial cells) are observed in the models of the invention, whereas rather moderate damages are observed in a conventional model (Figs. 8 to 11 of Example 5 and Figs. 16 & 17 of Comparative Example 3);
(4) the model animals of the invention (model animal examples 1 to 3) can be prepared in about 20 minutes, whereas a conventional model animal (comparative model animal example 1) requires 3 hours or longer to be prepared; and
(5) model animals having the corneal epithelial damages with a severity enough to be used for evaluating the therapeutic effects of medicines (models of the present invention) can not be prepared by using a material which simply absorbs water without generating the difference in osmotic pressure between the inside and outside of the corneal epithelium cells (for example, a water-absorbing material) as a material to be contacted with the ocular surface for the dehydration in preparing a model animal (see Comparative Examples 2 and 4).

### INDUSTRIAL APPLICABILITY

The model animal of the present invention is useful as a model of a corneal epithelial damage. Particularly, the model animal of the present invention is useful as a model of a corneal epithelial damage caused by a disorder of the lacrimal fluid, or so-called dry-eye model. Further, the model animal of the present invention is useful for the method of preparing a model animal of the present invention and the method of evaluating a medicine using the model animal of the present invention.

As the model animal of the present invention, 1) a variety of model animals having different severities of symptom can be prepared by adjusting the conditions such as the kind of the water-absorbing material, the size of contact area and the contact time. Moreover, the model animal of the present invention has excellent features that, for instance, 2) the degree of action of a test medicine can be determined quantitatively, which a conventional model animal does not have.

The method of preparing a model animal of the present invention has excellent features that a model animal having a given severity can be constantly produced in a short period of time.

Further, the evaluation method of the present invention has excellent features that, for instance, 1) the effect of a medicine can be evaluated with an excellent reproducibility; 2) the effect of a medicine can be determined quantitatively; 3) since it is also easy to quantitatively compare the therapeutic effect of a test medicine with that of a standard medicine(comparing in terms of ED5o or the like), the method of the present invention is also useful as a screening system of new medicines, which a conventional model animal does not have.

## Claims

1. A model animal having a corneal epithelial damage, **characterized in that** the corneal epithelial damage is caused by contacting an ocular cornea of said animal with a water-absorbing material and thereby generating the difference in osmotic pressure between the inside and outside of corneal epithelium cells.

2. The model animal according to Claim 1, **characterized in that** said corneal epithelial damage is dry eye.

3. The model animal according to Claim 1 or 2, **characterized in that** said model animal is a non-human mammalian or a fowl.

4. The model animal according to any one of Claims 1 to 3, **characterized in that** the water-absorbing material comprises at least one of materials selected from a polyol, a salt, an amino acid, a peptide and a water-soluble synthetic polymer.

5. The model animal according to Claim 4, **characterized in that** the water-absorbing material is a saccharide.

6. The model animal according to Claim 5, **characterized in that** the water-absorbing material comprises at least one of saccharides selected from the group consisting of glucose, maltose, sucrose, fructose, dextran and starch.

7. The model animal according to Claim 4, **characterized in that** the water-absorbing material comprises at least one of salts selected from an alkali metal salt and an alkaline earth metal salt.

8. The model animal according to any one of Claims 1 to 7, **characterized in that** the water-absorbing material is in the form of any one of powder, solution, gel, jelly and tablet.

9. The model animal according to any one of Claims 1 to 8, **characterized in that** the water-absorbing material is contacted with the ocular cornea through a water-permeable or semi-permeable product.

10. The model animal according to any one of Claims 1 to 9, **characterized in that** the model animal is rabbit.

11. The model animal according to any one of Claims 1 to 10, **characterized in that** the water-absorbing material is contacted with a portion of the ocular cornea.

12. The model animal according to any one of Claims 1 to 11, **characterized in that** the water-absorbing material is contacted with a pupil area of the ocular cornea.

13. The model animal according to Claim 12, **characterized in that** the water-absorbing material is contacted with the pupil area of the ocular cornea so that the contact area becomes a round shape.

14. The model animal according to Claim 13, **characterized in that** contact of the water-absorbing material is carried out by covering the ocular cornea with a water-impermeable thin membrane having a hole in its center part to place the hole on the pupil area, and contacting the water-absorbing material with the exposed site.

15. A method of evaluating a medicine for treatment of a corneal epithelial damage, **characterized in that** the method comprises the steps of:
contacting an ocular cornea of a model animal with a water-absorbing material and thereby generating the difference in osmotic pressure between the inside and outside of corneal epithelium cells to produce a symptom of the corneal epithelial damage;
administering a test medicine to the damaged ocular cornea; and
evaluating the degree of the symptom ameliorated in the ocular corneal epithelial damage site.

16. The method according to Claim 15, **characterized in that** said corneal epithelial damage is dry eye.

17. The method according to Claim 15 or 16, **characterized in that** the step of evaluating the degree of the symptom ameliorated in the corneal epithelial damage site is a step of evaluation by expressing the change in the stained area in terms of numerical values.

18. The method according to any one of Claims 15 to 17, **characterized in that** the model animal according to any one of Claims 1 to 14 is used.

19. The method according to any one of Claims 15 to 18, **characterized in that** the test medicine is an eye drop.

20. A medicine for treatment of a corneal epithelial damage, which is selected by the method according to any one of Claims 15 to 19.
